# EUROPEAN PATENT APPLICATION

(11) **EP 4 285 901 A1**
(43) Date of publication of application: **06.12.2023**
(21) Application number: 21923380.6
(22) Date of filing: 28.10.2021
(51) Int. Cl.: A61K 31/445, A61K 31/198, A61P 9/10, A61P 25/28, A23L 33/10

(54) **COMPOSITE PHARMACEUTICAL COMPOSITION FOR TREATMENT OF BRAIN DISEASE, COMPRISING CHOLINESTERASE INHIBITOR AND ANTIOXIDANT**

(30) Priority: 28.01.2021 KR 20210012659
(71) Applicant: Dr.Noah Biotech Inc., Suwon-si, Gyeonggi-do 16229 (KR)
(72) Inventor: LEE, Ji Hyun, Suwon-si, Gyeonggi-do 16229 (KR); KIM, Eun Jung, Suwon-si, Gyeonggi-do 16229 (KR)
(74) Representative: Richardt Patentanwälte PartG mbB
(86) International application number: PCT/KR2021/015350
(87) International publication number: WO 2022/163971

(57) **Abstract**

The present invention relates to a composite pharmaceutical composition for the treatment of brain disease, comprising a cholinesterase inhibitor and an antioxidant, and more specifically to a composition for the prevention or treatment of brain disease, comprising: a cholinesterase inhibitor exhibiting a synergistic effect in neuronal protection and the promotion of neuronal differentiation; and an antioxidant.

## Description

### TECHNICAL FIELD

This application claims priority to Korean Patent Application No. 10-2021-0012659 filed on January 28, 2021, and the entire specifications of which are incorporated herein by reference in their entireties.

The present invention relates to a composite pharmaceutical composition for the treatment of brain disease, comprising a cholinesterase inhibitor and an antioxidant, and more specifically to a composition for the prevention or treatment of brain disease, comprising: a cholinesterase inhibitor exhibiting a synergistic effect in neuronal protection and the promotion of neuronal differentiation; and an antioxidant.

### BACKGROUND OF THE INVENTION

According to data released by Statistics Korea in 2010, the number of elderly people aged 65 and over accounted for 11.4% of the total population in 2011, and is expected to reach 37.4% by 2050, making Korea an ultra-aging society. As the aging problem has become a social issue in recent years, the public's interest in the characteristics of the elderly population and the welfare of the elderly, including housing, health, culture, and leisure, is increasing, and the demand for statistics is also increasing.

At the heart of this shift is the growing aging population, which is making chronic degenerative diseases a bigger problem than acute infectious diseases, which have been the leading cause of death for the past 50 years. Among chronic degenerative disorders, cerebrovascular disease is a very important one, ranking second in terms of single-cause mortality.

These cerebrovascular diseases can be categorized into two main types. One is hemorrhagic brain disease, which is caused by a cerebral hemorrhage, and the other is ischemic brain disease, which is caused by a blockage in the blood vessels of the brain. Hemorrhagic brain disease is most often caused by traffic accidents, while ischemic brain disease is more common in the elderly.

In the event of a temporary cerebral ischemia, the supply of oxygen and glucose to the brain is cut off, causing neurons to experience reduced ATP and edema, leading to extensive brain damage. Neuronal death occurs a significant amount of time after cerebral ischemia, which is called delayed neuronal death. Delayed neuronal death has been reported in experiments using a transient forebrain ischemic model in the Mongolian gerbil, where neuronal death is observed in the CA1 region of the hippocampus 4 days after induction of 5 minutes of cerebral ischemia (Kirino T, Sano K. Acta Neuropathol., 62: 201-208, 1984; Kirino T. Brain Res., 239: 57-69, 1982).

There are two most widely recognized mechanisms of neuronal death from cerebral ischemia. One is the excitatory neuronal death mechanism, in which cerebral ischemia causes excessive extracellular glutamate to accumulate, and this increased glutamate causes neuronal death due to excessive intracellular calcium accumulation (Kang TC, et al., J. Neurocytol., 30: 945-955, 2001), the other is oxidative neuronal death, which is induced by damage to DNA and cytoplasm due to an increase in free radicals caused by sudden oxygenation during ischemia-reperfusion (Won MH, et al., Brain Res., 836: 70-78,1999; Sub AY., Chen YM., J. Biomed. Sci., 5: 401-414, 1998; Flowers F, Zimmerman JJ. New Horiz. 6: 169-180, 1998).

Based on these mechanistic studies, many studies have been conducted to identify substances that effectively inhibit neuronal death during cerebral ischemia or to elucidate their mechanisms. However, there are still few substances that effectively inhibit neuronal death induced by cerebral ischemia.

Tissue plasminogen activator, the only FDA-approved treatment for cerebral ischemia to date, is a thrombolytic, meaning it dissolves the blood clot that causes cerebral ischemia, allowing for a faster supply of oxygen and glucose. Therefore, it does not directly protect neurons, so it needs to be used quickly, and because it is a thrombolytic agent, if it is used in excess or frequently, the blood vessel wall becomes thinner and eventually causes hemorrhagic cerebrovascular disease. In addition, in the case of MK-801, a calcium channel blocker to effectively inhibit early calcium influx, clinical tests were conducted, but the drug was discarded due to its side effects. In Japan, Mitsubishi's edaravone, a synthetic antioxidant, is prescribed only in Japan as the only drug in the world for the treatment of severe gout due to successful clinical trials in Japan, with annual sales of 300 billion won, but it can be said that it is being used due to the lack of a drug despite concerns about side effects.

On the other hand, since cognitive dysfunctions such as Alzheimer's disease occur mainly in the elderly population over the age of 60, research on the pathogenesis of these diseases is essential in view of the aging of the Korean population, which is progressing at a very fast pace, and the serious psychological distress and economic burden it can cause not only to the patients themselves, but also to their families, society, and the country. In addition, such research requires the urgent development of effective drugs for the prevention or improvement of diseases at the present time, which are only able to improve symptoms or slow down the development of diseases.

The causes of Alzheimer's disease can be divided into external and internal factors, external factors include age, genetic causes, and toxic substances caused by environmental influences, internal factors include decreased neurotransmission due to decreased acetylcholine concentration, neuroinflammation due to cytokines, cytotoxicity due to aggregation of amyloid-beta and hyperphosphorylated tau protein, and neuronal death due to oxidative stress. Neuronal death has been pointed out as a fundamental pathology common to many brain diseases, and the factors and molecular mechanisms that trigger cell death have been studied in depth. However, previously developed proteolytic enzymes and broad-spectrum apoptosis inhibitors suffer from side effects and decreased clinical effectiveness.

### DETAILED DESCRIPTION OF THE INVENTION

### TECHNICAL PROBLEM

Accordingly, the present inventor conducted a preliminary study to develop a synergistic therapeutic combination for the treatment of ischemic brain diseases and neurodegenerative diseases among the clinically safe disclosed substances, and found that the combination of donepezil and N-acetylcysteine exhibited significantly enhanced neuroprotective and differentiation-promoting effects compared to each drug alone, and completed the present invention.

Accordingly, it is an object of the present invention to provide a pharmaceutical composition for preventing or treating ischemic brain disease comprising a cholinesterase inhibitor and an antioxidant as active ingredients.

In addition, an object of the present invention is to provide a pharmaceutical composition for preventing or treating ischemic brain disease consisting of a cholinesterase inhibitor and an antioxidant.

In addition, an object of the present invention is to provide a pharmaceutical composition for preventing or treating ischemic brain disease essentially consisting of a cholinesterase inhibitor and an antioxidant.

Another object of the present invention is to provide a pharmaceutical composition for preventing or treating neurodegenerative diseases comprising a cholinesterase inhibitor and an antioxidant as active ingredients.

In addition, an object of the present invention is to provide a pharmaceutical composition for preventing or treating neurodegenerative diseases consisting of a cholinesterase inhibitor and an antioxidant.

In addition, an object of the present invention is to provide a pharmaceutical composition for preventing or treating neurodegenerative diseases essentially consisting of a cholinesterase inhibitor and an antioxidant.

Another object of the present invention is to provide a food composition for preventing or improving ischemic brain disease comprising a cholinesterase inhibitor and an antioxidant as active ingredients.

In addition, an object of the present invention is to provide a food composition for preventing or improving ischemic brain disease consisting of a cholinesterase inhibitor and an antioxidant.

In addition, an object of the present invention is to provide a food composition for preventing or improving ischemic brain disease essentially consisting of a cholinesterase inhibitor and an antioxidant.

Another object of the present invention is to provide a food composition for preventing or improving neurodegenerative diseases comprising a cholinesterase inhibitor and an antioxidant as active ingredients.

In addition, an object of the present invention is to provide a food composition for preventing or improving neurodegenerative diseases consisting of a cholinesterase inhibitor and an antioxidant.

In addition, an object of the present invention is to provide a food composition for preventing or improving neurodegenerative diseases essentially consisting of a cholinesterase inhibitor and an antioxidant.

Another object of the present invention is to provide a method for treating ischemic brain disease in animals other than humans, comprising administering a cholinesterase inhibitor and an antioxidant simultaneously, separately or sequentially.

Another object of the present invention is to provide a method for treating neurodegenerative diseases in animals other than humans, comprising administering a cholinesterase inhibitor and an antioxidant simultaneously, separately or sequentially.

Another object of the present invention is to provide use of a cholinesterase inhibitor and an antioxidant for the preparation of agents for the treatment of ischemic brain disease.

Another object of the present invention is to provide a method for treating ischemic brain disease comprising administering an effective amount of a composition comprising a cholinesterase inhibitor and an antioxidant to a subject in need thereof.

Another object of the present invention is to provide use of a cholinesterase inhibitor and an antioxidant for the preparation of agents for the treatment of neurodegenerative diseases.

Another object of the present invention is to provide a method for treating neurodegenerative diseases comprising administering an effective amount of a composition comprising a cholinesterase inhibitor and an antioxidant to a subject in need thereof.

### TECHNICAL SOLUTION

In order to achieve the above object of the present invention, the present invention provides a pharmaceutical composition for preventing or treating ischemic brain disease comprising a cholinesterase inhibitor and an antioxidant as active ingredients.

In addition, the present invention provides a pharmaceutical composition for preventing or treating ischemic brain disease consisting of a cholinesterase inhibitor and an antioxidant.

In addition, the present invention provides a pharmaceutical composition for preventing or treating ischemic brain disease essentially consisting of a cholinesterase inhibitor and an antioxidant.

In order to achieve the above object of the present invention, the present invention provides a pharmaceutical composition for preventing or treating neurodegenerative diseases comprising a cholinesterase inhibitor and an antioxidant as active ingredients.

In addition, the present invention provides a pharmaceutical composition for preventing or treating neurodegenerative diseases consisting of a cholinesterase inhibitor and an antioxidant.

In addition, the present invention provides a pharmaceutical composition for preventing or treating neurodegenerative diseases essentially consisting of a cholinesterase inhibitor and an antioxidant.

In order to achieve the above object of the present invention, the present invention provide a food composition for preventing or improving ischemic brain disease comprising a cholinesterase inhibitor and an antioxidant as active ingredients.

In addition, the present invention provides a food composition for preventing or improving ischemic brain disease consisting of a cholinesterase inhibitor and an antioxidant.

In addition, the present invention provides a food composition for preventing or improving ischemic brain disease essentially consisting of a cholinesterase inhibitor and an antioxidant.

In order to achieve the above object of the present invention, the present invention provide a food composition for preventing or improving neurodegenerative diseases comprising a cholinesterase inhibitor and an antioxidant as active ingredients.

In addition, the present invention provides a food composition for preventing or improving neurodegenerative diseases consisting of a cholinesterase inhibitor and an antioxidant.

In addition, the present invention provides a food composition for preventing or improving neurodegenerative diseases essentially consisting of a cholinesterase inhibitor and an antioxidant.

In order to achieve the above object of the present invention, the present invention provide a method for treating ischemic brain disease in animals other than humans, comprising administering a cholinesterase inhibitor and an antioxidant simultaneously, separately or sequentially.

In order to achieve the above object of the present invention, the present invention provide a method for treating neurodegenerative diseases in animals other than humans, comprising administering a cholinesterase inhibitor and an antioxidant simultaneously, separately or sequentially.

In order to achieve the above object of the present invention, the present invention provide use of a cholinesterase inhibitor and an antioxidant for the preparation of agents for the treatment of ischemic brain disease.

In order to achieve the above object of the present invention, the present invention provide a method for treating ischemic brain disease comprising administering an effective amount of a composition comprising a cholinesterase inhibitor and an antioxidant to a subject in need thereof.

In order to achieve the above object of the present invention, the present invention provide use of a cholinesterase inhibitor and an antioxidant for the preparation of agents for the treatment of neurodegenerative diseases.

In order to achieve the above object of the present invention, the present invention provide a method for treating neurodegenerative diseases comprising administering an effective amount of a composition comprising a cholinesterase inhibitor and an antioxidant to a subject in need thereof.

Hereinafter, the present invention will be described in detail.

The present invention provides a pharmaceutical composition for preventing or treating ischemic brain disease comprising a cholinesterase inhibitor and an antioxidant as active ingredients.

The present invention provides a pharmaceutical composition for preventing or treating neurodegenerative diseases comprising a cholinesterase inhibitor and an antioxidant as active ingredients.

In the present invention, the "cholinesterase inhibitor" refers to a compound that inhibits the enzymatic degradation of the neurotransmitter acetylcholine and thus increases the duration and level of acetylcholine action in the synaptic cleft. Two enzymes are primarily responsible for the breakdown of acetylcholine, acetylcholinesterase and butyrylcholinesterase. The "cholinesterase inhibitor" comprises substances that inhibit or otherwise reduce the action of one or both of these enzymes.

In the compositions and methods of the present invention, cholinesterase inhibitors are considered pharmaceutically effective. As used herein, "pharmaceutically effective" means that the cholinesterase inhibitor is therapeutically useful in humans. For this reason, the term excludes cholinesterase inhibitors used as pesticides, such as aldicarb (2-methyl-2-(methylthio)propionaldehyde O-methylcarbamoyloxime), carbofuran (2,3-dihydro-2,2-dimethyl-7-benzofuranyl methylcarbamate), and carbaryl (1-naphthyl methylcarbamate), and cholinesterase inhibitors lethal enough to humans to be used as chemical weapons, such as sarin (2-(fluoro-methylphosphoryl)oxypropane), VX (S-[2-(diisopropylamino)ethyl ]-O-ethyl methylphosphonothioate), and soman (3-(fluoro-methyl-phosphoryl)oxy-2,2-dimethylbutane). Most cholinesterase inhibitors, pesticides and chemical weapons, are quasi-reversible or irreversible; Most of the pharmaceutically effective cholinesterase inhibitors are reversible.

Pharmaceutically effective cholinesterase inhibitors are well known in the art. For example, 7-methoxytacrine, albameline, ambenonium, anseculin, arecoline, cevimeline, citicoline, demacarium, donepezil, edrophonium, eptastigmine, fasciculin, heptyl-physostigmine, huperzine A and its analogs, icopezil , ipidacrine, linopiridine, metrifonate, milameline, neostigmine, neostigmine, pyridostigmine, norpyridostigmine, tacrine, physostigmine, rivastigmine, subcomeline, suronacrine, tacrine analogues, tacrine, talsaclidine, velnacrine, xanomeline, zifrosilone, itopride, acotiamide, huperzine, galanthamine and salts thereof may be included, but not limited thereto, and most preferably, the cholinesterase inhibitor may be donepezil.

Donepezil is an acetylcholinesterase (AChE) inhibitor having a structure represented by Formula 1 below and is used for the treatment of mild to moderate dementia in Alzheimer's disease. In Alzheimer's disease, in which cholinergic nervous system disorders in the brain have been reported, donepezil activates cholinergic neurons in the brain by increasing acetylcholine in the brain. Donepezil currently used commercially is in the form of a tablet (pill), and is being prescribed to patients with Alzheimer's disease in the form of an oral drug.

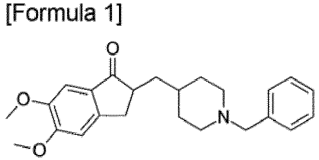

As the pharmaceutically acceptable salt of donepezil, an acid addition salt formed with a pharmaceutically acceptable free acid is useful. Acid addition salts are obtained from inorganic acids such as hydrochloric acid, nitric acid, phosphoric acid, sulfuric acid, hydrobromic acid, hydroiodic acid, nitrous acid or phosphorous acid and non-toxic organic acids such as aliphatic mono- and dicarboxylates, phenyl-substituted alkanoates, hydroxy alkanoates and alkanedioates, aromatic acids, aliphatic and aromatic sulfonic acids. These pharmaceutically non-toxic salts include sulfate, pyrosulfate, bisulphate, sulphite, bisulfite, nitrate, phosphate, monohydrogen phosphate, dihydrogen phosphate, metaphosphate, pyrophosphate chloride, bromide, ioda Id, fluoride, acetate, propionate, decanoate, caprylate, acrylate, formate, isobutyrate, caprate, heptanoate, propiolate, oxalate, malonate, succinate, suberate , sebacate, fumarate, maleate, butyne-1,4-dioate, hexane-1,6-dioate, benzoate, chlorobenzoate, methylbenzoate, dinitro benzoate, hydroxybenzoate, toxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycol Late, maleate, tartrate, methanesulfonate, propanesulfonate, naphthalene-1-sulfonate, naphthalene-2-sulfonate or mandelate may be used, but is not limited thereto.

In the present invention, the "antioxidant" is a substance that helps protect the human body from oxidative stress by removing active oxygen generated in the body, and its type is well known in the art. For example, the antioxidant may include N-acetylcysteine, ascorbic acid, alpha-lipoic acid, scopoletin, forsythin, isoferulic acid, gamma-oryzanol, trans-anethol, thioctic acid, cysteamine, gallic acid, salvianolic acid, vitamin E, lappaconitine, L-selenomethionine, selenium, coenzyme Q10, vitamin A, catechin, daltro etc., but is not limited thereto, preferably, it may be N-acetylcysteine, alpha-lipoic acid, daltron or a salt thereof, most preferably, it may be N-acetylcysteine or a salt thereof.

The N-acetylcysteine is a drug for inhibiting oxidative stress having the structure of Formula 2 below, which was developed about 50 years ago and is mainly used as an expectorant or used for the treatment of acetaminophen poisoning, which is an analgesic. N-acetylcysteine is known to exhibit antioxidant effects by directly reacting with peroxide, hydrogen peroxide, hydroxyl radical, etc. to reduce oxidative stress or indirectly increasing glutathione synthesis by providing cysteine, a raw material for glutathione biosynthesis. N-acetylcysteine has been used for decades in various clinical fields such as treatment of acute respiratory distress syndrome, treatment of acute pulmonary oxygen toxicity, and prevention of acute renal failure by contrast agents, and is known as a safe drug that does not cause side effects.

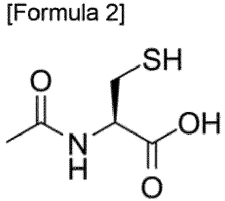

According to one embodiment of the present invention, it was confirmed that the combination agent comprising donepezil and N-acetylcysteine had remarkably superior neuronal cell protection and differentiation promoting effects compared to each single substance.

Therefore, the composition of the present invention comprising donepezil and N-acetylcysteine can exhibit more improved effects with a lower dose compared to using each drug alone, so it has the advantage of reducing side effects and improving the treatment effect of brain diseases, that is, the synergic effect.

The bliss independence combined response C for two single compounds with effects A and B is C = A + B - A*B, where each effect is expressed as a fractional inhibition value from 0 to 1 (see Bliss (1939) Annals of Applied Biology). The Bliss value, defined as the difference between the experimental response and the calculated independent Bliss value, indicates whether the effect of the two components combined is additive or synergistic.

A bliss value of zero (0) is considered additive. The term "additive" means that the result of combining two types of target agents is the sum of each of the individual drugs.

The term "synergy" or "synergistic" is used to mean that the reaction of the combination of the two agents is greater than the sum of the reactions of the individual agents. More particularly, in an in vitro setting, one measure of synergy is known as "Bliss synergy". The Bliss synergy means "exceeding the Bliss independent value" determined by the previously defined Bliss value. If the bliss value is greater than zero, it is considered an indicator of synergy. Of course, the concept of "synergy" as used in the present invention comprises in vitro synergy measured by additive and/or alternative methods.

In the present invention, the fact that the biological effect (including but not limited to anti-inflammatory effects) of the combination of the cholinesterase inhibitor and the antioxidant in vitro is greater than or equal to the sum of the individual components of the combination may be correlated with the Bliss value. In addition, "synergy", which includes cases where a combination of components exhibits an activity equal to or greater than the sum of the individual components, as used herein, can be measured by additional and/or alternative methods.

In one embodiment of the present invention, the composition of the present invention is for treating brain diseases, an effective amount of a cholinesterase inhibitor or a pharmaceutically effective salt, derivative or metabolite thereof can be combined with an effective amount of an antioxidant or pharmaceutically effective salt thereof in an amount sufficient to achieve a synergistic effect.

In one embodiment of the invention, the cholinesterase inhibitor and the antioxidant may be combined in a molar ratio of 1:0.1 to 2000, preferably in a molar ratio of 1:1 to 1000, more preferably in a molar ratio of 1:50 to 500, even more preferably in a molar ratio of 1:100 to 400, and most preferably in a molar ratio of 1:100 to 350.

In the present invention, the ischemic brain disease is also called cerebrovascular disease, and any pathological abnormality in the blood vessels supplying blood to the brain due to thrombosis, embolism, cerebrovascular thickening, cerebrovascular occlusion, etc. may be included without limitation. Non-limiting examples of the ischemic brain disease may include stroke, cerebral hemorrhage, cerebral infarction, head trauma, cerebral circulatory metabolic disorder, vascular dementia, and cerebral functional coma.

In the present invention, the neurodegenerative disease may be selected from the group consisting of Alzheimer's disease, Parkinson's disease, dementia, cognitive dysfunction, progressive supranuclear palsy, multiple system atrophy, olive-pontine-cerebellar atrophy (OPCA), Shy-Drager syndrome, striatal-substantia nigra degeneration, Huntington's disease, amyotrophic lateral sclerosis (ALS), essential tremor, cortico-basal ganglia degeneration, diffuse Lewy body disease, Parkinson-ALS-dementia complex, Niemann-Pick disease and Pick disease, but is not limited thereto.

The cognitive dysfunction, as it is deeply related to aging, may include without limitation any disease in which abnormal nerve cell death occurs rapidly in a part of the nervous system or in the entire brain, resulting in loss of function of the brain and spinal cord, thereby reducing cognitive ability, unlike the normal aging process. Non-limiting examples of the cognitive dysfunction may include mild cognitive impairment, Alzheimer's disease, frontotemporal dementia, Lewy body disease, cortico-basal ganglia degeneration, learning disabilities, agnosia, amnesia, aphasia, apraxia and delirium.

The "prevention" means any action that reduces the frequency or severity of pathological phenomena. Prevention can be complete or partial. In this case, it may mean a phenomenon in which the symptoms of carcinogenesis in the subject are reduced compared to the case where the composition is not used.

The "treatment" refers to any act that intervenes clinically to change the natural process of a target or cell to be treated, it can be performed while the clinical pathology is progressing or to prevent it.

A desired therapeutic effect may include preventing occurrence or recurrence of a disease, alleviating the symptoms, reducing any direct or indirect pathological consequences of the disease, reducing the rate of disease progression, alleviating or temporarily relieving a disease condition, or improving prognosis.

In the compositions of the present invention, the donepezil and N-acetylcysteine may be administered simultaneously, separately or sequentially, and the donepezil and N-acetylcysteine may act jointly in the body to exhibit brain disease prevention or treatment activity.

Typically, the cholinesterase inhibitor and antioxidant will be administered simultaneously as a composition, however, even if each of the active agents is administered to the human body at a staggered time, an equivalent level of therapeutic activity may be achieved by the simultaneous action of each of the active ingredients administered separately in the body.

Specifically, "administered simultaneously" means administering the two active ingredients together via the same route of administration, or administering them separately via the same or different routes of administration at substantially the same time (e.g., with a time interval of 15 minutes or less between administrations). Administered separately means that the two active ingredients are administered via the same or different routes of administration at a certain time interval (e.g., 3 days apart). Sequentially means administering the two active ingredients through the same or different routes of administration with a certain preceding and following rule, depending on the disease state of the patient.

The route of administration may be oral or parenteral. Parenteral administration may include, but is not limited to, intravenous, intramuscular, intraarterial, intramedullary, intrathecal, intrathecal, intracardiac, transdermal, subcutaneous, intrathecal, intracerebroventricular (subventricular zone), intracerebral, intraperitoneal, intranasal, enteral, topical, sublingual, or rectal.

A pharmaceutical composition according to the invention may comprise a pharmaceutically effective amount of donepezil and N-acetylcysteine alone, or may additionally comprise a pharmaceutically acceptable carrier. By "pharmaceutically effective amount" is meant an amount that produces a reaction that is greater than or equal to that of a negative control, preferably an amount sufficient to increase lifespan, improve motility, inhibit neuroinflammation, inhibit neuronal cell death, or promote neuronal cell differentiation by co-administration of the two active ingredients in treating or preventing brain disease.

The pharmaceutical compositions of the present invention can be formulated in a variety of ways, depending on the route of administration, in conjunction with a pharmaceutically acceptable carrier, by methods known in the art to produce the synergistic effects of combining a cholinesterase inhibitor and an antioxidant. By "pharmaceutically acceptable" is meant a composition that is physiologically acceptable and, when administered to humans, non-toxic, which does not interfere with the action of the active ingredient and does not normally cause gastrointestinal disturbances, allergic reactions such as dizziness, or similar reactions. The carriers include solvents of all kinds, dispersion media, oil-in-water or oil-in-water emulsions, aqueous compositions, liposomes, microbeads and microsomes.

Pharmaceutically acceptable carriers for inclusion in the compositions are those conventionally utilized in pharmaceutical formulations, including, but not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil. Other pharmaceutically acceptable carriers are known in the art.

In addition to the above ingredients, the pharmaceutical composition may further comprise lubricants, wetting agents, sweeteners, flavoring agents, emulsifiers, suspending agents, preservatives, and the like. Specifically, for oral administration, a binder, an active agent, a disintegrant, an excipient, a solubilizer, a dispersant, a stabilizer, a suspending agent, a color or flavor, and the like may be used, for injectables, a mixture of buffers, preservatives, non-currency agents, solubilizers, isotonic agents, stabilizers, and the like may be used, for topical administration, a base, excipients, lubricants, preservatives, etc. may be used.

In addition, the compositions of the present invention can be used in the form of conventional pharmaceutical preparations. Parenteral preparations include sterile aqueous solutions, non-aqueous solutions, suspensions, emulsions or lyophilized preparations, injections, transdermal preparations, nasal inhalations, and the like, for oral administration, it may be formulated as tablets, troches, capsules, elixirs, suspensions, syrups, or wafers. In the case of injectables, they can be prepared in single-dose ampoules or multiple-dose formulations. In the case of injectables, they must be sterile and protected from contamination by microorganisms such as bacteria and fungi. Examples of suitable carriers for injectables include, but are not limited to, solvents or dispersion media comprising water, ethanol, polyols (e.g., glycerol, propylene glycol, and liquid polyethylene glycol), mixtures thereof, and/or vegetable oils. More preferably, suitable carriers include Hanks' solution, Ringer's solution, phosphate buffered saline (PBS) containing triethanolamine, or isotonic solutions such as sterile water for injection, 10% ethanol, 40% propylene glycol, and 5% dextrose. To protect the injectable from microbial contamination, the injectable may additionally contain various antibacterial and antifungal agents such as parabens, chlorobutanol, phenols, sorbic acid, thimerosal, and the like. In addition, the injectable may further comprise an isotonic agent such as sugar or sodium chloride in most cases.

Furthermore, the pharmaceutical compositions of the present invention may be administered by any device that allows the active substance to be transported to the target site. Preferred modes of administration and formulations include intravenous, subcutaneous, intradermal, intramuscular, or drip injections. The injectable formulation can be prepared using an aqueous solvent, such as saline or intravenous solution, a non-aqueous solvent, such as a vegetable oil, a higher fatty acid ester (e.g., oleic acid ethyl), an alcohol (e.g., ethanol, benzyl alcohol, propylene glycol, or glycerin), etc, and included in pharmaceutical carriers such as stabilizers to prevent spoilage (e.g., ascorbic acid, sodium hydrogen sulfite, sodium pyrosulfite, BHA, tocopherols, EDTA, etc.), emulsifiers, buffers to adjust pH, and preservatives to inhibit microbial growth (e.g., phenylmercury nitrate, thimerosal, benzalkonium chloride, phenol, cresol, benzyl alcohol, etc.). A method of treating or preventing a neurodegenerative disease using a composition of the present invention comprises administering an effective amount (pharmaceutically effective amount) of a therapeutic composition of the present invention to a subject in need thereof. The pharmaceutically effective amount can be readily determined by those skilled in the art based on factors well known in the medical field, such as the type of disease, the patient's age, weight, health, gender, the patient's sensitivity to the drug, the route of administration, the method of administration, the number of doses, the duration of treatment, and the drugs used in combination or concurrently.

In addition, the pharmaceutical compositions of the present invention can be formulated using methods known in the art to provide a rapid, sustained, or delayed release of the active ingredient after administration to a mammal.

The total effective amount of a composition of the present invention may be administered to a patient in a single dose, or in a fractionated treatment protocol where multiple doses are administered over an extended period of time. The pharmaceutical compositions of the present invention can have different amounts of the active ingredient depending on the severity of the disease. Preferred overall doses of the pharmaceutical compositions of the invention may be from about 0.01 µg to 10,000 mg per kilogram of patient body weight per day, most preferably from 0.1 µg to 500 mg. However, it should be noted that the effective dose of the pharmaceutical composition to a patient will be determined not only by the method of formulation, route of administration, and number of treatments, but also by a variety of factors, including the age, weight, health status, gender, severity of the disease, diet, and excretion rate of the patient, and in light of these considerations, one having ordinary skill in the art will be able to determine the appropriate effective dose of the composition of the invention. The pharmaceutical compositions according to the present invention are not particularly limited in their formulation, route of administration and method of administration as long as they exhibit the effects of the present invention.

Depending on the method and route of administration, the compositions of the present invention may be formulated so that the components donepezil and N-acetylcysteine are simultaneously included in one formulation, or each component may be formulated separately and included in one package according to the unit of administration, such as daily or once daily. The separately formulated dosage forms of donepezil and N-acetylcysteine may or may not be identical. Specific methods of formulating the pharmaceutical compositions of the present invention, as well as pharmaceutically acceptable carriers that may be included in the formulations, are as described above for the pharmaceutical compositions, with reference to those known in the art.

The present invention also provides a food composition for preventing or improving ischemic brain disease, comprising a cholinesterase inhibitor and an antioxidant as active ingredients.

The present invention also provides a food composition for preventing or improving neurodegenerative diseases comprising a cholinesterase inhibitor and an antioxidant as active ingredients.

The food compositions of the present invention include all forms of functional foods, nutritional supplements, health foods, and food additives.

Food compositions of the above types can be prepared in various forms according to conventional methods known in the art. By way of example, but not limitation, the cholinesterase inhibitor and antioxidant can be liquefied, granulated, encapsulated, and powdered for consumption in the form of teas, juices, and drinks. In addition, donepezil and N-acetylcysteine can be formulated into compositions by mixing them with the active ingredients of the disclosure known to be effective against infectious diseases. Furthermore, In addition, cholinesterase inhibitors and antioxidants can be added to beverages (including alcoholic beverages), fruits and their processed products (e.g., canned, bottled, jam, marmalade, etc.), fish, meat and their processed products (e.g., ham, sausage corned beef, etc.), breads and noodles (e.g., udon, soba, ramen, spaghetti, macaroni, etc.), fruit juices, various beverages, cookies, malt, dairy products (e.g., butter, cheese, etc.), edible vegetable oils, margarine, vegetable proteins, retort foods, frozen foods, various condiments (e.g., miso, soy sauce, sauces, etc.), etc. including, but not limited to, functional foods. In addition, cholinesterase inhibitors and antioxidants can be formulated in powder or concentrate form for use as additives.

Preferred amounts of donepezil and N-acetylcysteine in the food compositions of the present invention include, but are not limited to, 0.1 to 90 wt% of the final prepared food product. More preferably, food compositions comprising the cholinesterase inhibitors and antioxidants of the present invention as active ingredients can be prepared in the form of a health functional food or dietary supplement, particularly in combination with active ingredients known to be effective against infectious diseases.

The present invention also provides a method for treating ischemic brain disease in animals other than humans, comprising administering a cholinesterase inhibitor and an antioxidant simultaneously, separately or sequentially.

The present invention also provides a method for treating neurodegenerative diseases in animals other than humans, comprising administering a cholinesterase inhibitor and an antioxidant simultaneously, separately or sequentially.

The present invention provides use of a cholinesterase inhibitor and an antioxidant for the preparation of agents for the treatment of ischemic brain disease.

The present invention provides a method for treating ischemic brain disease comprising administering an effective amount of a composition comprising a cholinesterase inhibitor and an antioxidant to a subject in need thereof.

The present invention provides use of a cholinesterase inhibitor and an antioxidant for the preparation of agents for the treatment of neurodegenerative diseases.

The present invention provides a method for treating neurodegenerative diseases comprising administering an effective amount of a composition comprising a cholinesterase inhibitor and an antioxidant to a subject in need thereof.

The 'effective amount' of the present invention refers to an amount that, when administered to a subject, exhibits an effect of improving, treating, preventing, detecting, diagnosing, or inhibiting or reducing ischemic brain disease and neurodegenerative diseases, the 'subject' may be an animal, preferably an animal, including a mammal, particularly a human, and may be an animal-derived cell, tissue, organ, or the like. The subject may be a patient in need of the effect.

The 'treatment' of the present comprehensively refers to improving ischemic brain disease and neurodegenerative diseases or the symptoms of the diseases, which may include curing, substantially preventing, or ameliorating the condition of ischemic brain disease and neurodegenerative diseases, and include alleviating, curing or preventing one or most of the symptoms resulting from the disease, but is not limited thereto.

In the present invention, the term 'comprising' is used synonymously with 'including' or 'characterized', and in the composition or method, additional component elements or method steps not mentioned are not excluded. The term 'consisting of' means excluding additional elements, steps, or ingredients not otherwise specified. The term 'essentially consisting of means, in the scope of the composition or method, including the described component elements or steps as well as the component elements or steps that do not substantially affect the basic properties thereof.

### ADVANTAGEOUS EFFECT

The composition according to the present invention can exhibit increased preventive and therapeutic effects of ischemic brain disease and neurodegenerative diseases compared to single administration by administering a cholinesterase inhibitor and an antioxidant in combination, it has the effect of reducing side effects that may be caused by excessive administration or long-term administration of each drug.

### BRIEF DESCRIPTION OF THE DRAWINGS

FIG. 1 is the result of evaluating the amount of reduction in active oxygen (antioxidant effect) after treatment with donepezil alone, N-acetylcysteine (NAC) alone, or donepezil + NAC combination (NDC-002) after inducing oxidative stress in nerve cells (SH-SY5Y).
FIG. 2 is a result of evaluating the anti-inflammatory effect by measuring the level of Interleukin-6 (IL-6) generated after single or combined treatment of donepezil or NAC together with lipopolysaccharide (LPS), which is an inflammatory response-inducing substance, in microglial cells (BV2 cell).
FIG. 3 is a result of measuring the concentration of NO generated after treatment with donepezil alone, NAC alone, or donepezil + NAC combination with LPS in microglia.
FIG. 4 is a result of evaluating the anti-inflammatory effect of NAC-like drugs by measuring the amount of NO generated after combined treatment of donepezil and antioxidant-based drugs with LPS to microglia cells.
FIG. 5 is a result of evaluating the cytotoxicity of NAC-like drugs by measuring the number of viable cells after combined treatment of donepezil and antioxidant-based drugs with LPS to microglial cells.
FIG. 6 is a result of measuring NO concentration generated after treatment with donepezil alone, antioxidant-based drug alone, or donepezil + antioxidant-based drug combination with LPS in microglia.
FIG. 7 is a result of evaluating the expression level of HB9, a motor neuron marker, after treatment with donepezil alone, NAC alone, or donepezil + NAC combination in neural progenitor cells (ReNcell VM).
FIG. 8 is the result of evaluating the differentiation promoting effect and cytotoxicity of donepezil-like drugs by measuring the expression level of HB9, a motor neuron marker, and the number of living cells after combined treatment of cholinesterase inhibitor drug and NAC in neural progenitor cells.
FIG. 9 is a result of evaluating the differentiation promoting effect of NAC-like drugs by measuring the expression level of HB9, a motor neuron marker, after combined treatment of donepezil and antioxidant-based drugs in neural progenitor cells.
FIG. 10 is a result of evaluating the cytotoxicity of NAC-like drugs by measuring the number of viable cells after a combined treatment of donepezil and an antioxidant-based drug in neural progenitor cells.
FIG. 11 is a result of measuring the expression difference of PSD-95 and pCREB involved in the regulation of synaptic plasticity after treatment with donepezil alone, NAC alone, or donepezil + NAC combination in primary cultured neurons from mouse fetal brains.
FIG. 12 is a result of evaluating the motor function recovery effect of the composite compound compared to the single compound through the recovery increase of the Rotarod latency test on day 1, 3,7,14, 21 after treatment with donepezil alone, NAC alone, or donepezil + NAC combination after induction of stroke-reperfusion animal model for 21 days.
FIG. 13 is the result of quantifying the degree of brain damage after treatment with donepezil alone, NAC alone, or donepezil + NAC combination immediately after induction of stroke-reperfusion animal model for 21 days.

### MODE FOR CARRYING OUT INVENTION

Hereinafter, the present invention is described in detail by the following embodiments. However, the following embodiments are intended to illustrate the invention, and the invention is not limited by them.

### Example 1: Antioxidant Effects of Donepezil + NAC Complex

In cerebrovascular disease, ischemic stroke causes a large amount of free radicals to be produced due to reduced blood flow to the ischemic area, resulting in the accumulation of oxidized metabolites. These free radicals can cause oxidative stress, which can affect many organs, including brain cells. To minimize the damage from free radicals, cells activate antioxidants and antioxidant enzymes to remove free radical intermediates and inhibit oxidative reactions to defend cells and the body.

In the present invention, neuronal cells (SH-SY5Y) were treated with H₂O₂ to induce oxidative stress, and reactive oxygen species (ROS) production was measured after treatment with control, donepezil alone (5uM), N-acetylcysteine (NAC) alone (5uM), and donepezil (2.5uM) + NAC (2.5uM) combination.

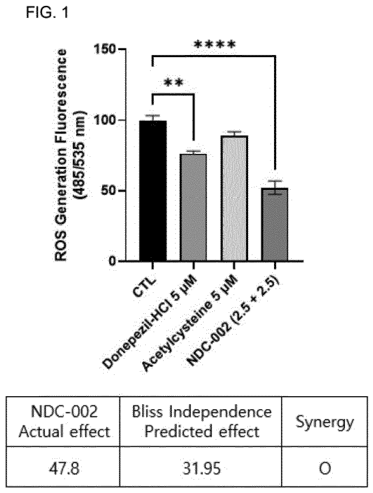

As a result, ROS were significantly reduced in the combined treatment group compared to the control group, as shown in FIG 1, and the combined treatment group showed a significantly enhanced reduction rate compared to the single treatment group of donepezil and NAC, indicating a synergistic effect of the combination on antioxidant effects (**P<0.01, *****P<0.0001, one-way ANOVA).*

### Example 2: Anti-inflammatory effect of donepezil + NAC complex

In the present invention, microglia (BV2 cells) were treated with LPS to generate an inflammatory response, and nitric oxide (NO) or inflammatory marker (IL-6) expression after treatment with donepezil alone, NAC alone, or combined donepezil + NAC was measured to determine the neuroinflammatory alleviating effects of the drug.

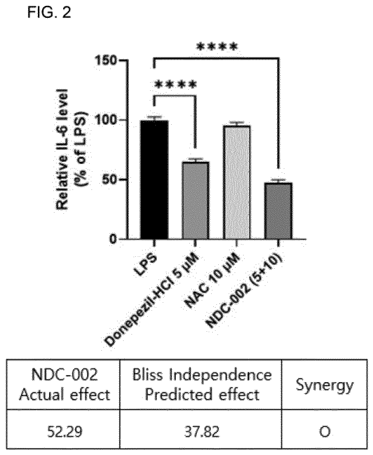

Microglia were treated with LPS to induce an inflammatory reaction and the expression of the inflammatory marker IL-6 was measured after treatment with donepezil alone, NAC alone, or donepezil + NAC combination, then the most significant anti-inflammatory effect was observed with the combination treatment of donepezil + NAC as shown in FIG. 2 (**** *P* < 0.0001, one-way ANOVA).

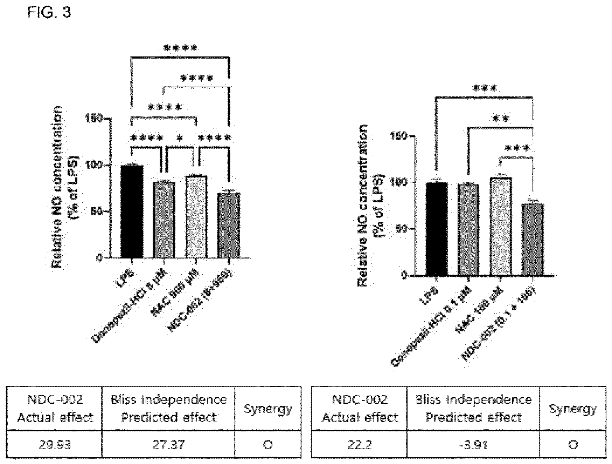

As shown in FIG. 3, it was quantified the reduction in NO production after treatment of microglia with LPS, after treatment with donepezil alone, NAC alone, or donepezil + NAC combination, confirming that donepezil + NAC combination exhibited significantly enhanced anti-inflammatory effects compared to the same concentration of single drug (**P* < 0.05, **P < 0.01, *** *P* < 0.001, **** *P* < 0.0001, one-way ANOVA).

To confirm whether the anti-inflammatory effect seen in the combined treatment of donepezil and NAC is also seen in the combination of donepezil, NAC and similar drugs, anti-inflammatory effects were confirmed at concentrations that did not show cytotoxicity in the drug combination of the similar drugs by measuring the amount of NO generated and the number of living cells after combination treatment of antioxidant drugs (22 types) similar to donepezil + NAC with LPS to microglia.

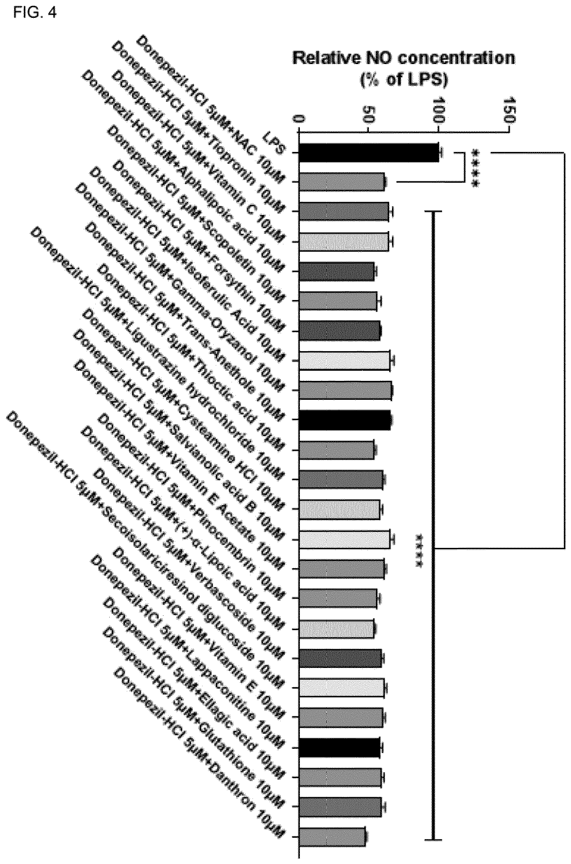

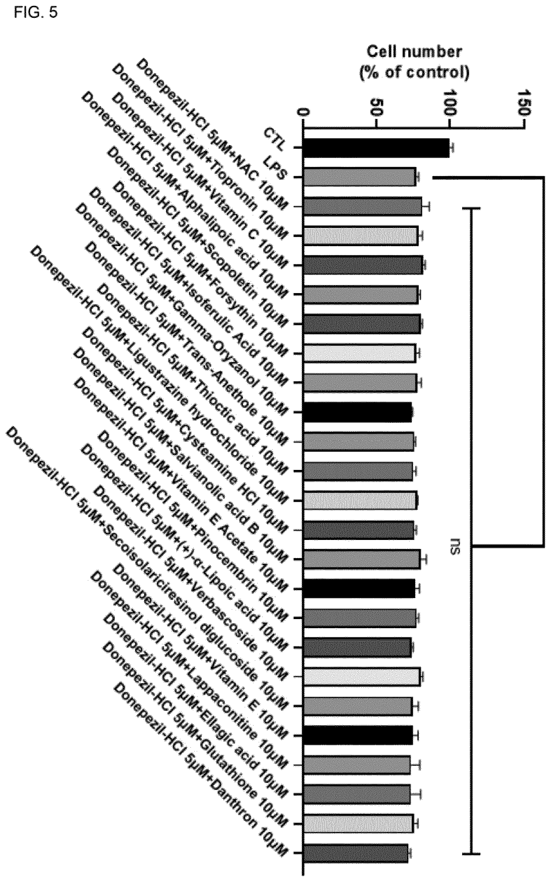

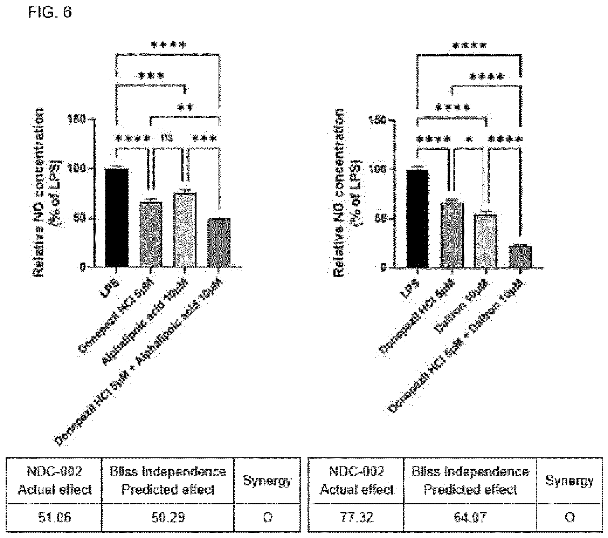

As a result, the donepezil + antioxidant combination treatment also showed a significant reduction in NO compared to the LPS alone group, as shown in FIGs 4 and 5, as shown in FIG. 6, the effect of donepezil + antioxidant combination treatment was confirmed to be synergistic compared to the same concentration of single drug (*P < 0.05, ***P* < 0.01, *** *P* < 0.001, **** *P* < 0.0001, one-way ANOVA).

### Example 3 : Neural progenitor cell differentiation effects of donepezil + NAC complex

ReNcell VMs (immortalized human neural progenitor cell line), neural progenitor cells that can differentiate into neurons and glial cells, were treated with donepezil alone, NAC alone, and donepezil + NAC combined, and then immunostained using an antibody that can selectively and specifically bind to motor neurons (anti-HB9 antibody). Afterwards, the number of motor neurons was determined by analyzing the level of HB9 positive signal, and the degree of differentiation of neural progenitor cells into motor neurons was measured.

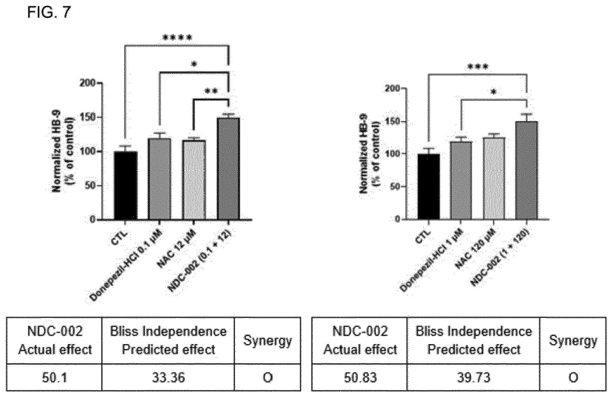

As a result, it was confirmed that the combined treatment group showed a synergistic effect in promoting neural progenitor cell differentiation compared to each treatment group of substance alone, as shown in FIG. 7 (**P* < 0.05, ***P* < 0.01, *** *P* < 0.001, **** *P* < 0.0001, one-way ANOVA).

To confirm whether there is differentiation ability into motor neurons in the case of combined treatment of acetylcholinesterase inhibitors other than donepezil + NAC and also in the case of combined administration of donepezil + antioxidants, nerve progenitor cells were treated with cholinesterase inhibitor drugs (8 types) similar to donepezil + NAC or antioxidant drugs (15 types) similar to donepezil + NAC, and the HB-9 level and the number of viable cells were measured, and the effect of promoting motor neuron cell differentiation and cytotoxicity of similar drug combinations were confirmed.

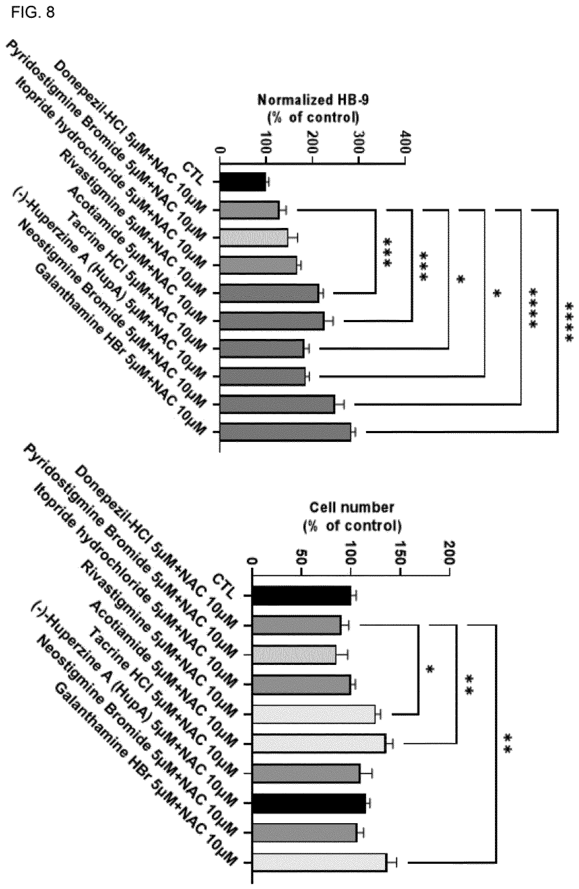

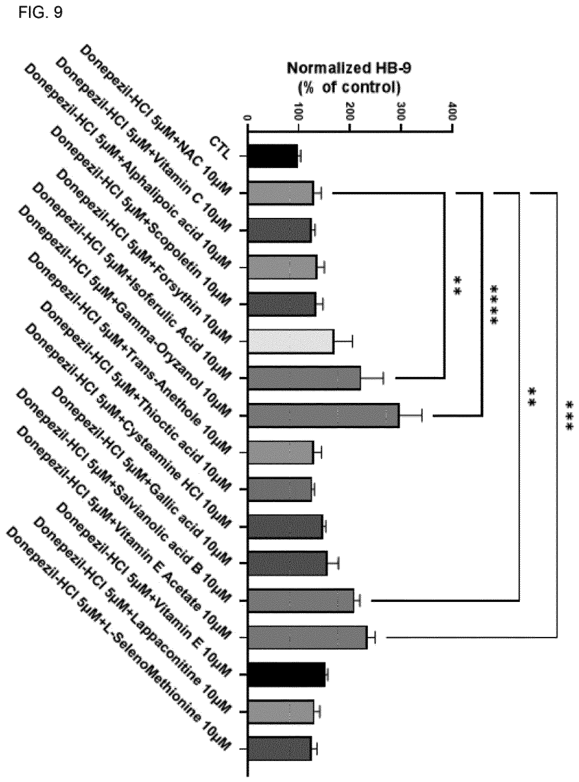

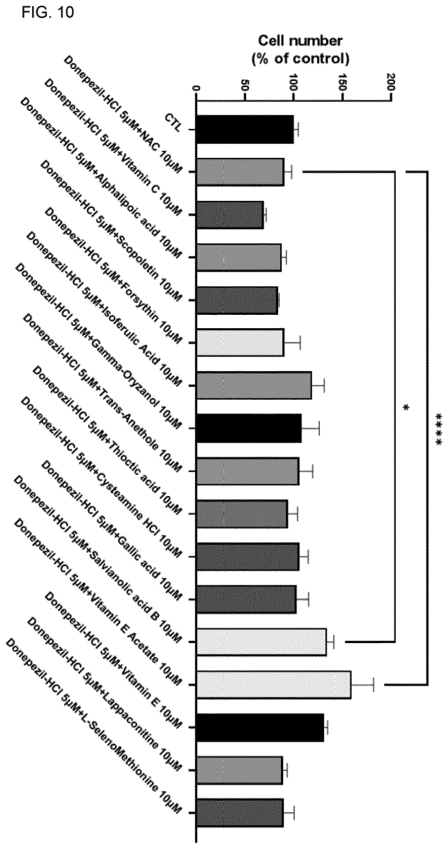

As a result, the combination treatment of 8 kinds of donepezil-like drugs + NAC showed a significantly increased differentiation promotion effect into motor neurons compared to the control group, as shown in FIG. 8. In addition, the combination treatment of 15 types of donepezil + NAC-like drugs, as shown in FIGs 9 and 10, also confirmed a significantly increased differentiation promotion effect into motor neurons compared to the control group, as seen in the combination treatment of donepezil + NAC (**P* < 0.05, ***P* < 0.01, *** *P* < 0.001, **** *P* < 0.0001, one-way ANOVA).

### Example 4: Synaptic plasticity enhancing effects of Donepezil + NAC Complex

Mouse primary neuron cultures were treated with donepezil alone, NAC alone, or combined donepezil + NAC and the expression of PSD-95 and pCREB, biomarkers of synaptic plasticity, were measured by Western blot assay.

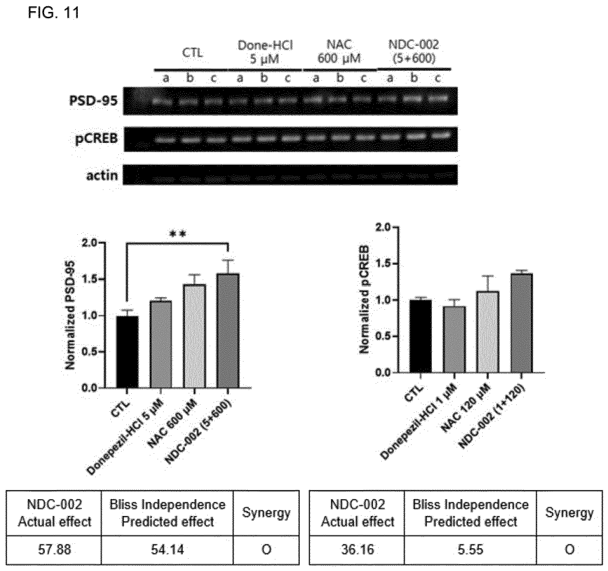

As a result, there was a synergistic trend in the increase in PSD-95 expression and the increase in pCREB in the donepezil + NAC combination treatment group compared to the control group, as shown in FIG. 11, confirming the synaptic plasticity enhancing effect of donepezil + NAC combination drug (** *P* < 0.01, one-way ANOVA).

For reference, type-specific biomarkers exist to identify cognitive and memory-enhancing functions. These include neuroprotective effects through inhibition of neuronal cell death, inhibition of beta amyloid formation and accumulation, inhibition of neuroinflammation, modulation of neurotransmitter production, secretion, and metabolism, and modulation of synaptic plasticity.

The regulation of synaptic plasticity is an important type of cognitive effect through its involvement in signaling between neurons, and its biomarkers include postsynaptic density-95 (PSD-95), CREB, and BDNF.

Postsynaptic density-95 (PSD-95) is an essential family of functional molecules that are part of the postsynaptic density (PSD) and play an essential role in the induction of long-term potentiation (LTP), including NMDA receptors and CaMKII. By binding directly or indirectly to neuroligins, NMDA receptors, AMPA receptors, and potassium channels, they play an important role in synaptic plasticity and the safety of synaptic changes during LTP (Ehrlich et al., PSD-95 is required for activity-driven synapse stabilization, PNAS 2007.1 Nagura et al., Impaired synaptic clustering of postsynaptic density proteins and altered signal transmission in hippocampal neurons, and disrupted learning behavior in PDZ1 and PDZ2 ligand binding deficient PSD-95 knockin mice, Molecular Brain, 2012.).

On the other hand, cAMP response element-binding protein (CREB) is a transcription factor that binds to promoter sites of various genes involved in memory and synaptic plasticity, and activation of CREB is known to induce transcription of genes involved in memory formation and consolidation (such as BDNF) (Mizuno et al., CREB phosphorylation as a molecular marker of memory processing in the hippocampus for spatial learning. Behavioural Brain Research, 2002. / Kida et al., Functional roles of CREB as a positive regulator in the formation and enhanced of memory, Brain Research Bulletin, 2014).

Therefore, it can be concluded that donepezil + NAC combination therapy, which synergistically increases the expression of PSD-95 and pCREB, will have a synergistic effect on improving cognition and memory.

### Example 5: Confirmation of synergistic effects of donepezil + NAC combination drug versus single in an in vivo experimental model

A stroke model was created using a cerebral infarction-reperfusion model in Sprague-Dawley rats (SD rats), and donepezil alone, NAC alone, and donepezil + NAC combination (NDC-002) were administered I.V. for 21 days immediately after the cerebral infarction-reperfusion model.

A representative test of motor coordination in an experimental animal model of stroke, the Rota-rod latency test, was used and animal motor function was measured on days 1, 3, 7, 14, and 21 after treatment with donepezil alone, NAC alone, and donepezil + NAC combination, and the rate of recovery of motor function was analyzed based on day 1 results.

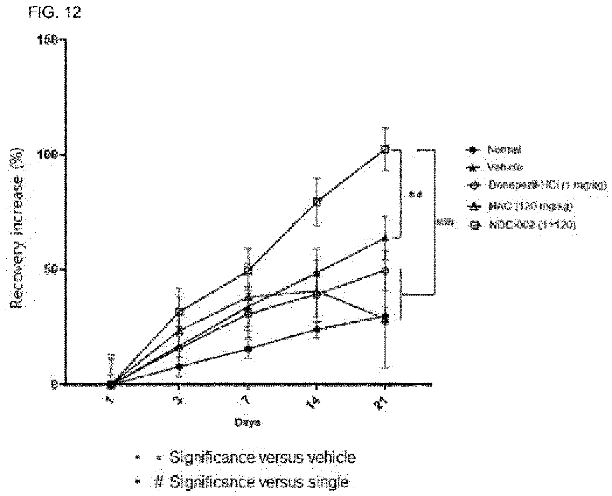

As a result, as shown in FIG. 12, it was confirmed that only the NDC-002 administration group showed a significant increase in recovery rate compared to the Vehicle administration group, showing the motor function recovery effect of the NDC-002 combination drug. (** *P* < 0.01, one-way ANOVA).

In addition, the recovery rate of motor function in the group administered with the donepezil + NAC combination drug showed a significant increase in the recovery rate compared to the group administered with the donepezil alone or the NAC alone, confirming the synergistic effect in the recovery of motor function of the NDC-002 combination drug (### *P* < 0.001 , one-way ANOVA).

As a result of analyzing the degree of brain damage by staining brain tissue with immunohistochemical staining using neurons (NeuN), microglia (GFAP) marker antibodies, and cell nucleus staining solution (DAPI) after administering donepezil alone, NAC alone, and donepezil + NAC combination drugs for 21 days immediately after stroke-reperfusion model (*P < 0.05, one-way ANOVA).

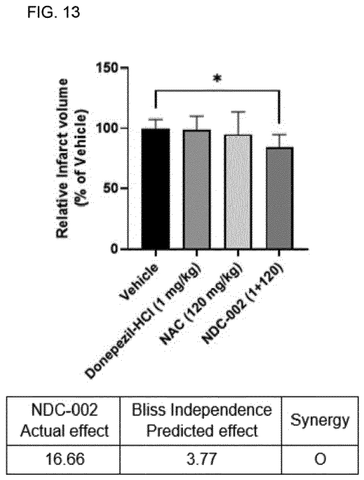

As shown in FIG. 13, it was confirmed that only the NDC-002 complex administration group showed a significant reduction in the degree of brain damage compared to the vehicle administration group.

### INDUSTRIAL APPLICABILITY

The compositions according to the present invention may exhibit enhanced preventive and therapeutic effects of brain diseases by co-administering a cholinesterase inhibitor and an antioxidant, compared to their single administration, and have the effect of alleviating side effects that may be caused by excessive dosage or long-term administration of each drug, therefore there are high industrial availability.

## Claims

1. A pharmaceutical composition for preventing or treating ischemic brain disease comprising a cholinesterase inhibitor and an antioxidant as active ingredients.

2. The pharmaceutical composition according to claim 1, wherein the ischemic brain disease is selected from the group consisting of stroke, cerebral hemorrhage, cerebral infarction, head trauma, cerebral circulatory metabolic disorder, vascular dementia, and cerebral functional coma.

3. The pharmaceutical composition according to claim 1, wherein the cholinesterase inhibitor is at least one selected from the group consisting of 7-methoxytacrine, albameline, ambenonium, anseculin, arecoline, cevimeline, citicoline, demacarium, donepezil, edrophonium, eptastigmine, fasciculin, heptyl-physostigmine, huperzine A and its analogs, icopezil , ipidacrine, linopiridine, metrifonate, milameline, neostigmine, nomeostigmine, pyridostigmine, norpyridostigmine, tacrine, physostigmine, rivastigmine, subcomeline, suronacrine, tacrine analogues, tacrine, talsaclidine, velnacrine, xanomeline, itopride, acotiamide, huperzine, galanthamine and ziprasidone or a salt thereof.

4. The pharmaceutical composition according to claim 1, wherein the antioxidant is at least one selected from the group consisting of N-acetylcysteine, ascorbic acid, alpha-lipoic acid, scopoletin, forsythin, isoferulic acid, gamma-oryzanol, trans -anethole, thioctic acid, cysteamine, gallic acid, salvianolic acid, vitamin E, lappaconitine, selenium, coenzyme Q10, vitamin A, catechin, daltron and L-selenomethionine or a salt thereof.

5. A pharmaceutical composition for preventing or treating neurodegenerative diseases comprising a cholinesterase inhibitor and an antioxidant as active ingredients.

6. The pharmaceutical composition according to claim 5, wherein the neurodegenerative disease is selected from the group consisting of Alzheimer's disease, Parkinson's disease, dementia, cognitive dysfunction, progressive supranuclear palsy, multiple system atrophy, olive-pontine-cerebellar atrophy (OPCA), Shy-Drager syndrome, striatal-substantia nigra degeneration, Huntington's disease, amyotrophic lateral sclerosis (ALS), essential tremor, cortico-basal ganglia degeneration, diffuse Lewy body disease, Parkinson-ALS-dementia complex, Niemann-Pick disease and Pick disease.

7. The pharmaceutical composition according to any one of claims 1 to 6, wherein the composition exhibits a neuronal differentiation promoting effect.

8. The pharmaceutical composition according to any one of claims 1 to 6, wherein the cholinesterase inhibitor and the antioxidant are administered simultaneously, separately or sequentially.

9. A food composition for preventing or improving ischemic brain diseases comprising a cholinesterase inhibitor and an antioxidant as active ingredients.

10. A food composition for preventing or improving neurodegenerative diseases comprising a cholinesterase inhibitor and an antioxidant as active ingredients.

11. The food composition according to claim 9 or 10, wherein the food is a health functional food.

12. A method for treating ischemic brain disease in animals other than humans, comprising administering a cholinesterase inhibitor and an antioxidant simultaneously, separately or sequentially.

13. A method for treating neurodegenerative disease in animals other than humans, comprising administering a cholinesterase inhibitor and an antioxidant simultaneously, separately or sequentially.

14. Use of a cholinesterase inhibitor and an antioxidant for the preparation of agents for the treatment of ischemic brain disease.

15. A method for treating ischemic brain disease comprising administering an effective amount of a composition comprising a cholinesterase inhibitor and an antioxidant to a subject in need thereof.

16. Use of a cholinesterase inhibitor and an antioxidant for the preparation of agents for the treatment of neurodegenerative diseases.

17. A method for treating neurodegenerative diseases comprising administering an effective amount of a composition comprising a cholinesterase inhibitor and an antioxidant to a subject in need thereof.
